# EUROPEAN PATENT APPLICATION

(11) **EP 4 678 767 A1**
(43) Date of publication of application: **14.01.2026**
(21) Application number: 24766563.1
(22) Date of filing: 08.03.2024
(51) Int. Cl.: C12Q 1/6883

(54) **USE OF MIRNAS AS BIOMARKERS OF SEVERE ALOPECIA AREATA**

(30) Priority: 09.03.2023 ES 202330196
(71) Applicant: Servicio Andaluz de Salud, 41071 Sevilla (ES); Universidad de Granada, 18071 Granada (ES)
(72) Inventor: RUANO RUIZ, Juan, 14004 Córdoba Córdoba (ES); GAY MIMBRERA, Jesús, 14004 Córdoba Córdoba (ES); GÓMEZ ARIAS, Pedro Jesús, 14004 Córdoba Córdoba (ES); LIÑARES BLANCO, José, 18071 Granada Granada (ES); ISLA TEJERA, Beatriz, 14004 Córdoba Córdoba (ES); AGUILAR LUQUE, Macarena, 14004 Córdoba Córdoba (ES)
(74) Representative: San Martin Alarcia, Esther
(86) International application number: PCT/ES2024/070150
(87) International publication number: WO 2024/184573

(57) **Abstract**

The invention related to the use of miRNAs as biomarkers in the prognosis and/or diagnosis of severe alopecia areata according to their expression levels. The invention also relates to methods for obtaining useful data for the diagnosis and/or prognosis of severe alopecia areata, as well as kits to carry out said methods.

## Description

### TECHNICAL FIELD

The present invention falls within the field of medicine, specifically the field of medical prognosis and diagnosis, and more specifically relates to the use of microRNAs (miRNAs) for the diagnosis and prognosis of severe alopecia areata.

### BACKGROUND OF THE INVENTION

Alopecia areata (AA) is an immune-mediated inflammatory skin disease that affects the hair follicle, characterized by non-scarring hair loss, usually in the form of single or multiple hairless plates measuring a few centimeters on the scalp. With a lifetime prevalence of 0.5-2%, it is estimated that the number of individuals affected by this disease in the US is 6.6 million and 147 million worldwide.

It usually appears in the form of mild cases that are controlled with topical treatments with corticoids. However, 10% of patients progress to severe forms of the disease, more extensives in the form of plates (>50% SALT), or to alopecia *totalis* (AT; total loss of hair on the scalp) or alopecia *universalis* (AU, loss of all body hair). Previous studies have shown that there is systemic inflammatory activity in the severe forms of the disease (Dubin C. et al. Scalp and serum profiling of frontal fibrosing alopecia reveals scalp immune and fibrosis dysregulation with no systemic involvement. J. Am. Acad. Dermatol. 2022 Mar;86(3):551-562. doi: 10.1016/j.jaad.2021.05.016. Epub 2021 May 24.).

Several drugs targeting severe cases are currently in clinical trials, so it would be interesting to know which patients are at greater risk of developing severe forms of the disease and, in such cases, to assess whether early therapies would be able to prevent this progression. To date, there are no methods for predicting the progression to these more aggressive forms of alopecia areata.

To date, clinical criteria have been used to establish the severity of alopecia areata, including the following:
- ***Severity of Alopecia Tool* (SALT),** combines the extent and density of hair loss on the scalp to provide an overall severity score for alopecia areata.
- **Alopecia Areata *Investigator Global Assessment* (AA-IGA)** is a visual scale that allows the severity of alopecia areata to be graded based on the patient's own inspection and that of the evaluating physician.
- **Alopecia Areata *Disease Activity Index* (ALADIN)** is a molecular activity index that uses a panel of genes expressed in the skin to classify the severity of patients and monitor the activity of alopecia areata. This group of genes is protected by a patent (US2019072541A1) described as an invention intended to improve the diagnosis and prognosis of the disease, capable of identifying subgroups of patients who respond to treatments, especially JAK kinase inhibitors.

The SALT and AA-IGA methods are based on the observation of the effects caused by the alopecia areata in patients, which means they have major limitations when it comes to predicting the progression of alopecia areata beyond establishing a scale for grading the disease at its current stage.

The ALADIN method proposes an alternative approach to the diagnosis and prognosis of the disease. However, it is a highly invasive methodology, as it requires a skin biopsy to obtain the skin samples object to study.

miRNAs are small endogenous non-coding RNAs approximately 18-25 nucleotides in length, capable of modulating the expression of complementary messenger RNAs. They down-regulate (inhibit) gene expression either by inhibiting translation or by cleaving the messenger RNA. Therefore, miRNAs are post-transcriptional regulators that bind to complementary sequences of mRNA transcripts by pairing with the untranslated region (3'-UTR), which generally results in repression of translation or degradation of the target, leading to gene silencing.

They are involved in the regulation of most molecular processes, and there is consistent scientific evidence regarding their relationship with various metabolic processes. Circulating miRNAs can be detected in the bloodstream, where they exhibit high stability and reproducibility. Circulating miRNAs can provide clinical information about pathophysiological conditions, enabling early diagnosis and the study of the progression of various diseases, therefore they are proposed as potential biomarkers.

Under a standard nomenclature system, experimentally confirmed miRNAs are assigned names as follows: the prefix "mir," followed by a hyphen and a number. The lowercase "mir-" refers to the pre-miRNA, while an uppercase "miR-" refers to the mature form. miRNAs with nearly identical sequences (except for one or two nucleotides) are annotated with an additional lowercase letter, e.g., miR-99a. Pre-miRNAs that lead to 100% identical mature miRNAs but are located at different sites in the genome are indicated with an additional hyphenated number suffix. The species of origin can be designated with a three-letter prefix, with hsa corresponding to a human miRNA *(Homo sapiens).* In the context of this document, where all individualized miRNAs are human miRNAs, the prefix "hsa-" is sometimes omitted. When two mature miRNAs originate from opposite arms of the same pre-miRNA, they are denoted with a suffix -3p or -5p, such as miR-142-3p. When relative expression levels are known, an asterisk after the name indicates a miRNA expressed at low levels relative to the miRNA on the opposite arm of a hairpin. miRNA sequences can be accessed at http://www.mirbase.org.

### BRIEF DESCRIPTION OF THE INVENTION

In the present invention, 19 miRNAs have been identified that act as biomarkers because they are differentially down-regulated in patients with severe alopecia areata.

Some of the deregulated miRNAs that make up this panel have been previously described in other dermatological diseases, but very few previous studies have analyzed the role of miRNAs in the peripheral blood of patients with alopecia areata, and those that address this aspect yield contradictory results. Of the 19 miRNAs proposed in the invention panel, only two have been previously described as associated with alopecia areata: miR-101-3p and miR-30b/c-5p. However, in that study, an increase in expression was found in the plasma of patients with alopecia areata and not a decrease, as described in the present invention.

The present invention **proposes the combined use of a series of microRNAs (miRNAs),** whose expression is decreased in the most severe forms of alopecia areata compared to mild forms or healthy individuals, as biomarkers for the diagnosis of the disease in severe stages. Differences in the expression of this group of miRNAs result in a deregulation of various immunological and metabolic pathways related to the severity of the disease.

The 19 miRNAs whose expression is decreased in the most severe forms of alopecia areata, or **miRNAs of the invention,** are as follows: miR-195-5p, miR-93-3p, miR-130b-3p, miR-21-3p, miR-214-3p, miR-101-3p, miR-153-3p, miR-16-5p, miR-296-5p, miR-325, miR-132-3p, miR-140-3p, miR-19a-3p, miR-29b-3p, miR-30b-5p, miR-30c-5p, miR-376a-3p, miR-424-5p, and miR-92a-3p. All of these are differentially down-regulated in patients with severe alopecia areata compared to patients with mild alopecia areata and controls, in whom no significant changes were found.

After validating these markers by RT-PCR in a different population, 14 of the 19 markers initially identified were identified as particularly relevant in terms of the degree of significance of the differences in expression between patients with severe alopecia areata and controls, specifically the following markers: miR 296-5p, miR 19a-3p, miR 30b-5p, miR 101-3p, miR 130b-3p, miR 30c-5p, miR 132-3p, miR 376a-3p, miR 29b-3p, miR 424-5p, miR 140-3p, miR 195-5p, miR 21-3p, miR 92a-3p.

Of these markers, 5 showed by themselves high sensitivity and specificity as biomarkers, both when used separately and in combination: miR 296-5p, miR 19a, miR 30b, miR 130-p, and miR 424-5p. These biomarkers are ranked below according to their sensitivity and specificity: miR 19a-3p, miR 130b-3p, miR 30b-5p, miR 296-5p, and finally, miR 424-5p. It should be noted that it is the combination of the 5 miRNAs mentioned above that shows the best results in terms of sensitivity and specificity.

The determination of the expression levels of the miRNAs of the invention is carried out in peripheral blood samples previously obtained from patients, preferably in plasma samples, making it minimally invasive compared to other diagnostic methods, such as the ALADIN index, which require a skin biopsy. Therefore, a method for diagnosing and predicting the progression of the disease to severe forms based on the determination of plasma expression levels of miRNAs is proposed below.

In turn, by measuring the expression levels of the miRNAs of the invention over time, this method is capable of monitoring the therapeutic response to treatment. The method of the invention can also be used as a molecular predictor of the therapeutic response to different treatments for alopecia areata. Thanks to the miRNAs proposed in the present invention and their differential expression in patients with mild and severe alopecia areata, it is possible to carry out an assessment and repositioning of drugs capable of reversing this state of deregulation. This assessment is performed using the Cmaps and LINCS tools. The Connectivity Map (CMap) project of the Broad Institute uses the library of integrated network-based cellular signatures (LINCS) and allows functional hypotheses to be generated from cellular signatures that represent systematic perturbations with genetic perturbagens (reflecting the function of proteins) and pharmacological perturbagens (reflecting the function of small molecules).

Once the lists of potential drugs on the market or in development or molecules in the experimental phase contained in these databases were obtained and after this analysis, 39 drugs potentially capable of reversing the state of deregulation in the expression of the miRNAs of the invention were finally selected. This is possible using GeneCodis, which uses the molecular signatures found to find similarities with other signatures produced by these perturbagens of interest and stored in the aforementioned databases. Finally, those results obtained with an FDR value < 0.05 and by the *relative enrichment* value in descending order were selected.

Of this final list, 41% of the drugs that showed a potential ability to reverse miRNA-mediated gene deregulation profiles in alopecia areata were kinase inhibitors (JAKinh) (sunitinib, pazopanib, semaxanib, sorafenib, dovitinib, tivozanib, axitinib, cediranib, orantinib, motesanib, linifanib, danusertib, ENMD-2076, SU-11652, BMS-536924, Tyrphostin-AG-1295, and PP30). Other drugs identified belonged to the antioxidant group (12.8%; catechin, curcumin, epicatechin, epigallocatechin, and ramnetin), epigenetic regulators (7.7%; azacitidine, zebularine), and antimicrobial agents (15.4%; triclosan, procainamide, pyrazinamide, flucytosine, triclabendazole, methylnorlichexanthone). 23.1% were substances with toxic effects or in very early stages of development, so they were not considered a priority.

Therefore, the present invention also develops a method capable of predicting the therapeutic response of different treatments, either under development or already approved for clinical use, against severe alopecia areata.

Finally, the present invention describes a kit for the analysis of miRNAs by RT-PCR using peripheral blood samples with the aim of classifying patients with alopecia areata according to their severity or prognosis into mild or severe forms.

### DETAILED DESCRIPTION OF THE INVENTION

**A first aspect** of the invention relates to the *in vitro* use of the expression levels of one or more of the following miRNAs: miR 19a-3p, miR 130b-3p, miR 30b-5p, miR 296-5p, and miR 424-5p as biomarkers for prognosing and/or diagnosing severe alopecia areata. In a preferred embodiment, the expression levels of these biomarkers are used simultaneously. In another preferred embodiment of this aspect of the invention, in addition to the expression levels of miR 19a-3p, miR 130b-3p, miR 30b-5p, miR 296-5p, and miR 424-5p, the expression levels of one or more of the following markers are also used: miR 101-3p, miR 30c-5p, miR 132-3p, miR 376a-3p, miR 29b-3p, miR 140-3p, miR 195-5p, miR 21-3p, and miR 92a-3p. In an even more preferred embodiment, the expression levels of these biomarkers are used simultaneously. In an even more preferred embodiment of this aspect of the invention, in addition to the expression levels of the aforementioned miRNAs, the expression levels of one or more of the following markers are used: miR-93-3p, miR-214-3p, miR-153-3p, miR-16-5p, and miR-325. In a preferred embodiment, the expression levels of all biomarkers in Table 1 are used simultaneously.

**Table 1: miRNA sequences of the invention.**

| **Name** | **SEQ ID No.** | **Sequence (5'-3')** | **Accession number** |
|---|---|---|---|
| hsa.miR-195-5p | 1 | uagcagcacagaaauauuggc | MIMAT0000461 |
| hsa.miR-93-3p | 2 | acugcugagcuagcacuucccg | MIMAT0004509 |
| **hsa.miR-130b-3p** | 3 | cagugcaaugaugaaagggcau | MIMAT0000691 |
| hsa.miR-21-3p | 4 | caacaccagucgaugggcugu | MIMAT0004494 |
| hsa.miR-214-3p | 5 | acagcaggcacagacaggcagu | MIMAT0000271 |
| hsa.miR-101-3p | 6 | uacaguacugugauaacugaa | MIMAT0000099 |
| hsa.miR-153-3p | 7 | uugcauagucacaaaagugauc | MIMAT0000439 |
| hsa.miR-16-5p | 8 | uagcagcacguaaauauuggcg | MIMAT0000069 |
| **hsa.miR-296-5p** | 9 | agggcccccccucaauccugu | MIMAT0000690 |
| hsa.miR-325 | 10 | ccuaguagguguccaguaagugu | MIMAT0000771 |
| hsa.miR-132-3p | 11 | uaacagucuacagccauggucg | MIMAT0000426 |
| hsa.miR-140-3p | 12 | uaccacaggguagaaccacgg | MIMAT0004597 |
| **hsa.miR-19a-3p** | 13 | ugugcaaaucuaugcaaaacuga | MIMAT0000073 |
| hsa.miR-29b-3p | 14 | uagcaccauuugaaaucaguguu | MIMAT0000100 |
| **hsa.miR-30b-5p** | 15 | uguaaacauccuacacucagcu | MIMAT0000420 |
| hsa.miR-30c-5p | 16 | uguaaacauccuacacucucagc | MIMAT0000244 |
| hsa.miR-376a-3p | 17 | aucauagaggaaaauccacgu | MIMAT0000729 |
| **hsa.miR-424-5p** | 18 | cagcagcaauucauguuuugaa | MIMAT0001341 |
| hsa.miR-92a-3p | 19 | uauugcacuugucccggccugu | MIMAT0000092 |

Another **aspect** of the invention relates to an expression profile of the miRNAs in Table 1, both individually and in any combination thereof, suitable for diagnosing severe alopecia areata, hereinafter referred to as the expression profile of biomarkers of the invention. Preferably, the expression profile is composed of one or more of the following miRNAs: miR 19a-3p, miR 130b-3p, miR 30b-5p, miR 296-5p, and miR 424-5p, more preferably all of them simultaneously, and even more preferably also includes one or more of the following markers: miR 101-3p, miR 30c-5p, miR 132-3p, miR 376a-3p, miR 29b-3p, miR 140-3p, miR 195-5p, miR 21-3p, and miR 92a-3p, more preferably all of them simultaneously, and even more preferably includes all the miRNAs in Table 1 simultaneously.

### METHODS OF THE INVENTION

Another **aspect** of the invention relates to an *in vitro* method for obtaining useful data for diagnosing and/or prognosing severe alopecia areata, comprising measuring the expression levels of one or more of the following miRNAs: miR 19a-3p, miR 130b-3p, miR 30b-5p, miR 296-5p, and miR 424-5p in a previously isolated biological sample. Preferably, the expression levels of all of them are measured simultaneously. Even more preferably, the expression levels of one or more of the following miRNAs are also measured: miR 101-3p, miR 30c-5p, miR 132-3p, miR 376a-3p, miR 29b-3p, miR 140-3p, miR 195-5p, miR 21-3p, and miR 92a-3p, more preferably, all of them simultaneously, and even more preferably, the expression levels of all the miRNAs in Table 1 are measured simultaneously.

Another **aspect** of the present invention relates to an *in vitro* method for diagnosing and/or prognosing severe alopecia areata comprising: measuring the expression levels of one or more of the miRNAs miR 19a-3p, miR 130b-3p, miR 30b-5p, miR 296-5p, and miR 424-5p in a previously isolated biological sample; comparing the values obtained with those of a reference sample obtained from healthy control individuals; and classifying the individual into the group of individuals suffering from severe alopecia areata when the miRNA(s) from step (a) are differentially down-expressed relative to the samples from healthy control individuals. Preferably, the expression levels of the 5 miRNAs are measured simultaneously. Even more preferably, the expression levels of one or more of the following markers are also measured: miR 101-3p, miR 30c-5p, miR 132-3p, miR 376a-3p, miR 29b-3p, miR 140-3p, miR 195-5p, miR 21-3p, and miR 92a-3p, more preferably, all of them simultaneously; and even more preferably, the expression levels of all the miRNAs in Table 1 are measured simultaneously.

In a preferred embodiment of this aspect of the invention, the biological sample is selected from blood, plasma, and serum. In an even more preferred embodiment, the biological sample is plasma.

The *in vitro* determination of expression levels is performed using any of the techniques known in the prior art. Preferably, the techniques are selected from the list consisting of a gene profiling method, such as a microarray, or a next-generation sequencing panel and/or a method comprising PCR, such as real-time PCR or RT-PCR; and/or Northern Blot; and/or an immunohistochemistry method; and/or an ELISA-based method.

A microarray is an array on a solid substrate (usually a glass slide or a silicon thin film cell) that analyzes large amounts of biological material, in this case a large number of different miRNAs or, preferably, their reverse DNA transcripts, which are detectable by specific probes immobilized on the solid substrate.

A Northern blot involves the use of electrophoresis to separate RNA samples by size and subsequent detection with a hybridization probe complementary to (part of) the target sequence of the RNA of interest.

Quantification of miRNA expression is preferably performed by RT-PCR. Reverse transcription PCR, or RT-PCR, allows the detection and amplification of RNA. RNA is reverse transcribed into complementary DNA (cDNA) using reverse transcriptase. The first step in RT-PCR is the synthesis of a DNA/RNA hybrid. Reverse transcriptase also has an RNase H function, which degrades the RNA portion of the hybrid. The single-stranded DNA molecule is then completed into cDNA by the reverse transcriptase activity of DNA-dependent DNA polymerase. From this point on, the standard PCR procedure is used to amplify the cDNA. The ability to reverse RNA into cDNA using RT-PCR has many advantages, as RNA is single-stranded and highly unstable, making it difficult to work with.

The method of the present invention can be applied to samples from individuals of any sex, i.e., male or female, and of any age.

In the method of the present invention, miRNA expression can be normalized, preferably in relation to the expression of another RNA molecule. There are well-known normalization methods in the prior art.

The invention provides a method for assigning a human subject to one of two groups: group 1, comprising subjects identifiable by the method of the invention, and group 2, representing the remaining subjects.

It is possible to provide personalized treatment to an individual depending on whether the individual is assigned to group 1 or group 2. Group 1 comprises subjects identifiable by the method of the invention as individuals suffering from severe alopecia areata, and group 2 represents the remaining subjects.

In another embodiment of the method of the invention, it is capable of predicting the therapeutic response of different treatments for severe alopecia areata, either in development or already approved for clinical use, based on their ability to reverse the state of deregulation in the expression of the miRNAs of the invention.

Therefore, we are talking about the use of one or more of the miRNAs, miR 19a-3p, miR 130b-3p, miR 30b-5p, miR 296-5p, and miR 424-5p, and preferably all of them simultaneously, in a method for predicting the therapeutic response of different treatments for severe alopecia areata. Even more preferably, one or more of the following markers are also used: miR 101-3p, miR 30c-5p, miR 132-3p, miR 376a-3p, miR 29b-3p, miR 140-3p, miR 195-5p, miR 21-3p, and miR 92a-3p, more preferably all of them simultaneously, and even more preferably all the miRNAs in Table 1 simultaneously.

Among the potential drugs that could be subjected to this assessment of therapeutic response, and therefore their suitability for the treatment of alopecia areata, kinase inhibitors stand out.

### KIT OR DEVICE OF THE INVENTION

**Another aspect** of the invention relates to a kit or device comprising at least one or more oligonucleotides capable of hybridizing with one or more of the miRNAs miR 19a - 3p, miR 130b-3p, miR 30b-5p, miR 296-5p, and miR 424-5p under stringent conditions, preferably with all of them simultaneously. Even more preferably, it comprises at least one or more oligonucleotides capable of hybridizing with one or more of the following miRNAs: miR 101-3p, miR 30c-5p, miR 132-3p, miR 376a-3p, miR 29b-3p, miR 140-3p, miR 195-5p, miR 21-3p, and miR 92a-3p, more preferably with all of them simultaneously, and even more preferably with all the miRNAs in Table 1 simultaneously.

It is preferred that said oligonucleotide(s) is capable of doing so under stringening conditions. In a preferred embodiment, one or more of said oligonucleotides (preferably DNA) are further defined by the following probes or primers: hsa-miRXX-miRCURY LNA miRNA measured with syber, specific LNA^{™} PCR primers. Stringency is a term used in hybridization experiments. Astringency reflects the degree of complementarity between the oligonucleotide and the nucleic acid (which in this case is the miRNA to be detected, e.g., hsa- ); the higher the astringency, the higher the percentage of homology between the probe and the nucleic acid bound to the filter. The person skilled in the art is well aware that temperature and salt concentrations have a direct effect on the results obtained. It is recognized that hybridization results are related to the number of degrees below the Tm (melting temperature) of the DNA in which the experiment is performed. Often, rigorous conditions are defined as washing with 0.1X SSC (sodium citrate saline (SSC) buffer at 65°C. (SSC is usually provided as a 20X stock solution, consisting of 3 M sodium chloride and 300 mM trisodium citrate (adjusted to pH 7.0 with HCl)).

In particular embodiments, the kit is selected from (a) a kit suitable for microarray analysis, (b) a kit suitable for PCR, (c) a kit suitable for Northern Blot, (d) a kit suitable for immunoassay. Two or more of these embodiments may also be combined, such that the kit may comprise, for example, both (a) and (c).

It is also possible that the oligonucleotide(s) are immobilized in spots on a surface (preferably solid). In one embodiment thereof, the kit comprises a microarray. An RNA microarray is an array on a solid substrate (usually a glass slide or a silicon thin film cell) that analyzes large amounts of different RNAs (in this case, miRNAs), which can be detected by specific probes immobilized at spots on the solid substrate. Each spot contains a specific nucleic acid sequence, typically a DNA sequence, known as probes (or informants). While the number of spots is not limited, there is a preferred embodiment in which the microarray is adapted to the methods of the invention. In one embodiment, a customized microarray of this type comprises fifty spots or fewer, such as thirty spots or fewer, including twenty spots or fewer.

In the case of (b) a kit suitable for PCR, this PCR is typically real-time quantitative PCR (RT-PCR), a sensitive and reproducible gene expression quantification technique. In this case, it is desirable that the kit additionally comprises a polyT oligonucleotide primer in addition to the kit oligonucleotide(s). The polyT oligonucleotide primer can be used together with the oligonucleotide(s) of the invention for priming PCR, after polyadenylation of the isolated miRNAs by methods known to those skilled in the art, such as the use of poly-(A) polymerase and ATP. These reagents may optionally be included in the kit.

A Northern blot involves the use of electrophoresis to separate RNA samples by size and subsequent detection with an oligonucleotide(s) (hybridization probe) complementary to (part of) the target sequence of the RNA of interest.

The kit or device of the invention may be used and the use is not particularly limited, although use in the method of the invention in any of its embodiments is preferred.

In another preferred embodiment of this aspect of the invention, the oligonucleotides, primers, probes, or antibodies are modified or labeled, for example, but without limitation, by radioactive or immunological labeling. Thus, preferably, the oligonucleotides have modifications in some of their nucleotides, such as, but not limited to, nucleotides having some of their atoms replaced with a radioactive isotope, typically 32P or tritium, nucleotides immunologically labeled, such as with a digoxigenin molecule, and/or immobilized on a membrane. Several possibilities are known in the prior art.

The kit or device of the invention may comprise controls, program instructions, and information necessary to carry out any of the methods of the invention.

Another **aspect** of the invention relates to the use of the kit according to any of the embodiments described above to carry out any of the embodiments of the method of the invention.

### COMPUTER-IMPLEMENTED INVENTION

Another **aspect** of the invention relates to computer-readable storage media comprising program instructions capable of causing a computer to perform the steps of any of the methods of the invention. The invention also extends to computer programs adapted for any processing medium to perform the methods of the invention. Such programs may take the form of source code, object code, an intermediate source code and object code, for example in partially compiled form, or in any other form suitable for use in implementing the processes according to the invention. The computer programs also encompass cloud-based applications based on this procedure. In particular, the invention encompasses computer programs arranged on or within a carrier. The carrier may be any entity or device capable of supporting the program. When the program is incorporated in a signal that can be carried directly by a cable or other device or medium, the carrier may consist of said cable or other device or medium. As a variant, the carrier could be an integrated circuit in which the program is included, the integrated circuit being adapted to execute, or to be used in the execution of, the corresponding processes.

For example, the programs could be incorporated into a storage medium, such as a ROM, a CD-ROM or a semiconductor ROM, a USB memory stick or a magnetic recording medium, for example, a floppy disk or a hard disk. Alternatively, the programs could be supported by a transmissible carrier signal. For example, it could be an electrical or optical signal that could be transported via an electrical or optical cable, by radio, or by any other means. The invention also extends to computer programs adapted so that any processing medium can carry out the methods of the invention. Such programs may take the form of source code, object code, an intermediate source code and object code, for example, in partially compiled form, or in any other form suitable for use in implementing the processes according to the invention. The computer programs also encompass cloud-based applications based on this procedure. Therefore, another aspect of the invention relates to a computer-readable storage medium comprising program instructions capable of causing a computer to perform the steps of any of the methods of the invention.

Another aspect of the invention relates to a transmissible signal comprising program instructions capable of causing a computer to perform the steps of any of the methods of the invention.

In the context of the present invention, the terms "subject," "patient," or "individual" are used here interchangeably to refer to all animals classified as mammals and include, but are not limited to, domestic and farm animals, primates, and humans, e.g., humans, non-human primates, cows, horses, pigs, sheep, goats, dogs, cats, or rodents. Preferably, the subject is a male or female human of any age or race.

Throughout the description and claims, the word "comprising" and its variants are not intended to exclude other technical features, additions, components, or steps. For those skilled in the art, other objects, advantages, and features of the invention will be understood in part from the description and in part from the practice of the invention. The following examples and drawings are provided for illustration purposes and are not intended to limit the present invention.

An "isolated biological sample" includes, but is not limited to, cells, tissues, and/or biological fluids from an organism, obtained by any method known to a person skilled in the art. Preferably, the isolated biological sample is peripheral venous blood.

The terms "one or more" or "at least one" or "several," as used herein, include one and the individualized specification of any number that is more than one, such as two, three, four, five, six, etc.

### DESCRIPTION OF FIGURES

**Figure 1****:** Expression values of the 17 miRNAs in Table 4, control vs. severe alopecia areata (AA).
**Figure 2****:** ROC curves based on bootstrap analysis with a reduced number of iterations (1000 iterations) for the 5 miRNAs (miR 19a-3p, miR 130b-3p, miR 30b-5p, miR 296-5p, miR 424-5p), showing the individual curves for each of them, the combination of all of them (combined), and some combinations in pairs.
**Figure 3****:** Distribution of prediction probabilities for alopecia areata with specific colors and shapes for the 5 miRNAs (miR 19a-3p, miR 130b-3p, miR 30b-5p, miR 296-5p, miR 424-5p), the combination of all of them (combined), and some combinations in pairs.
**Figure 4****:** Precision recall curve for the 5 miRNAs (miR 19a-3p, miR 130b-3p, miR 30b-5p, miR 296-5p, miR 424-5p), the combination of all of them (combined), and some combinations in pairs.
**Figure 5****:** Expression values of the 5 miRNAs (miR 19a-3p, miR 130b-3p, miR 30b-5p, miR 296-5p, miR 424-5p) for severe alopecia areata vs. vitiligo vs. control. FDR (p-value adjusted for false discovery rate). n.s. not significant. *p<0.05; **p<0.01; *** p<0.001.

### EXAMPLES OF THE INVENTION

The present invention is based on the analysis of 740 miRNAs in peripheral blood samples from patients with severe alopecia areata (n=12), mild alopecia areata (n=9), and healthy controls (n=5). After the approval of the protocol by the local Research Ethics Committee, patients were selected based on the following criteria: a) adults; b) with clinically or histologically diagnosed alopecia areata; c) unresponsive to local or systemic treatments or after drug washout (2 weeks for topical drugs and 4 weeks for non-biological systemic drugs); d) no other comorbidities or autoimmune diseases, except for Hashimoto's thyroiditis in an euthyroid state.

The severity of alopecia was assessed using the SALT scale, and the progression time of the disease was calculated based on each patient's medical history. Based on all these data, patients were classified into two groups: a) mild alopecia areata: plates with <50% extension and duration of less than one year; b) severe alopecia areata: duration greater than one year, ≥50% **SALT,** alopecia *totalis* (AT), or alopecia *universalis* (AU).

Once the various consents of the patient were obtained, photographs were taken, peripheral venous blood was drawn (DNA, plasma, RNAlatter, and Ficoll), and skin biopsies were obtained under local anesthesia with a 5 mm punch from the lesion area (LA) and non-lesion area (NL). In the case of healthy controls, a single sample was obtained from the retroauricular area of the scalp.

Peripheral venous blood was drawn after fasting and treated with anticoagulant (K2-EDTA 5.4 mg). To obtain plasma samples, it was centrifuged at 1800 g for 10 min at room temperature. The upper layer containing the plasma was stored at -80 °C until miRNA purification. The commercial Qiagen kit (miRNeasy Serum/Plasma Kit) was used for miRNA purification from plasma following the manufacturer's instructions. The miRNA was eluted with RNase-free water, quantified, and stored at -80 °C.

These samples were analyzed by RT-PCR using the OpenArray system and Thermofisher Connet. Expression values [cycle threshold (Ct)] were normalized with miRNA expression values, transforming Ct values to -dCt. Missing values were estimated for each gene as 20% of the minimum expression values in all samples. After performing quality control and batch correction, expression differences between groups were analyzed using a nonparametric approach with the Wilcoxon test and considering a significance level of p < 0.05. This yielded different ranked lists of differentially expressed miRNAs. Statistical analyses were performed using the statistical language R (www.r-project.org).

HGU133plus2.0 (Affymetrix) microarrays were used to measure gene expression. Preprocessing and statistical analysis of microarray data were performed using the statistical language R and Bioconductor. Harshlight was used for image quality control, and expressions were obtained using GCRMA. The p-values of the moderate (paired) t-test were adjusted for multiplicity using the Benjamini-Hochberg approach.

As a result, 19 differentially down-regulated miRNAs were identified in patients with severe alopecia areata compared to patients with mild alopecia areata and controls, in whom no significant changes were found (p<0.05): miR-195-5p, miR-93-3p, miR-130b-3p, miR-21-3p, miR-214-3p, miR-101-3p, miR-153-3p, miR-16-5p, miR-296-5p, miR-325, miR-132-3p, miR-140-3p, miR-19a-3p, miR-29b-3p, miR-30b-5p, miR-30c-5p, miR-376a-3p, miR-424-5p, and/or miR-92a-3p.

These miRNAs are identified in **Table 2,** indicating for each case the level of expression in patients with severe alopecia areata and in controls, as well as associated p-values.

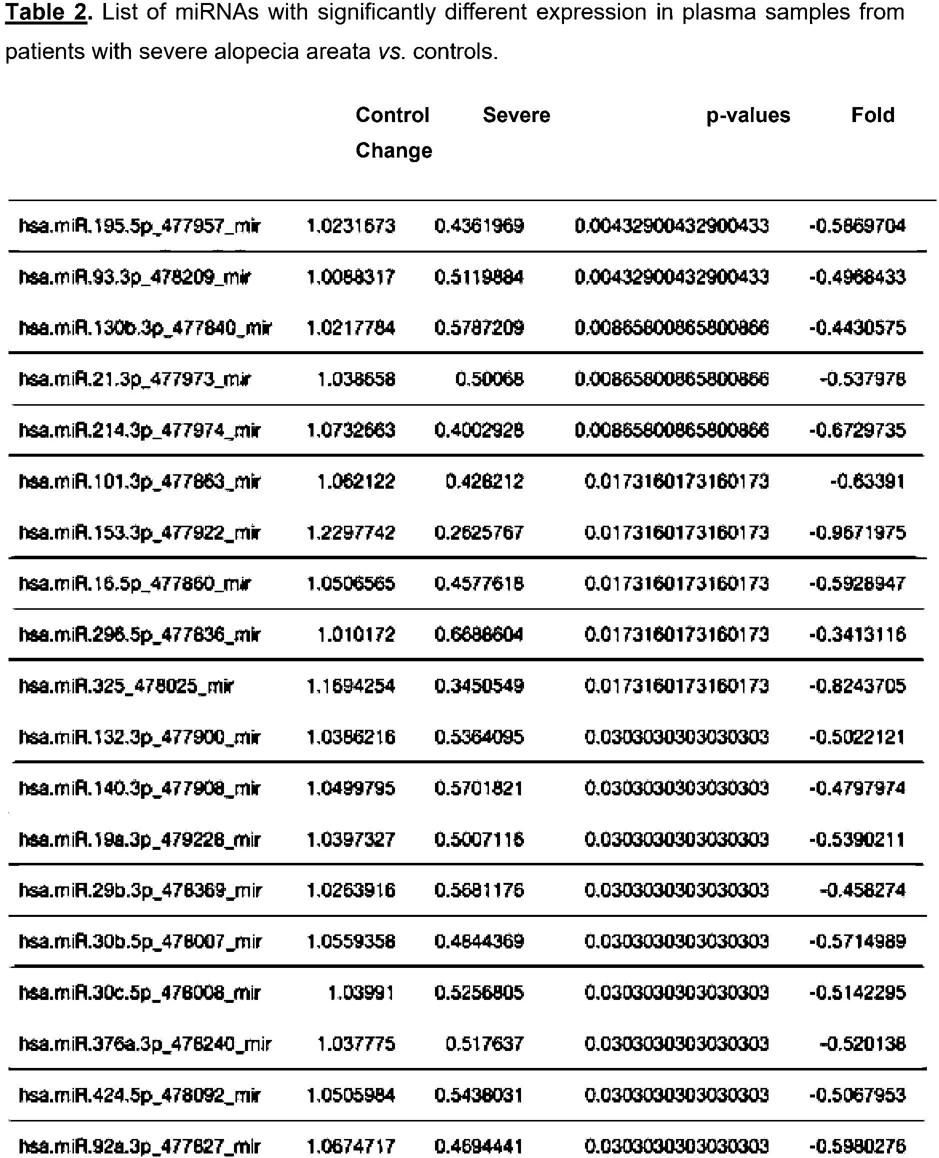

A series of miRNAs were also identified that were differentially expressed in plasma samples from patients with mild alopecia areata compared to controls. The results are shown below in Table 3 and correspond to the following miRNAs: miR-495-3p, miR-655-3p, and miR-187-3p. Two of these miRNAs were overexpressed and one was underexpressed.

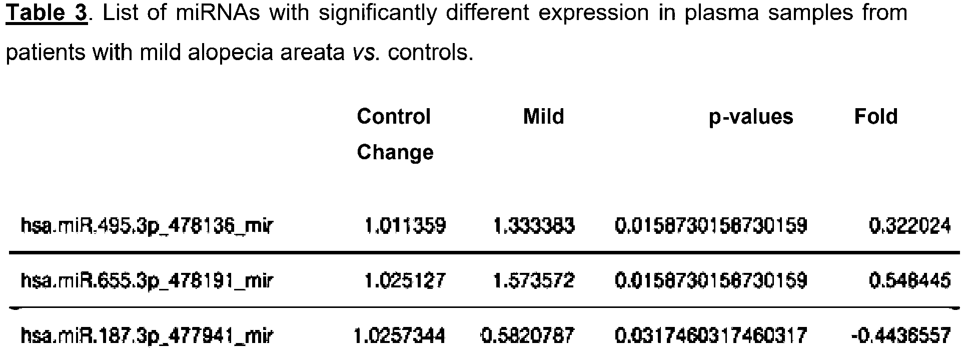

None of the 3 miRNAs deregulated in patients with mild alopecia areata were found in the panel of miRNAs underexpressed in severe alopecia areata in Table 1, confirming that the panel of miRNAs of the invention is selective in differentiating between different degrees of severity of alopecia areata.

### Validation of differentially expressed miRNAs in severe alopecia areata

The initial list of 19 miRNAs in Table 2 (miR-195-5p, miR-93-3p, miR-130b-3p, miR-21-3p, miR-214-3p, miR-101-3p, miR-153-3p, miR-16-5p, miR-296-5p, miR-325, miR-132-3p, miR-140-3p, miR-19a-3p, miR-29b-3p, miR-30b-5p, miR-30c-5p, miR-376a-3p, miR-424-5p, and miR-92a-3p) was validated by RT-PCR. From this list, miR-325 was used as a normalizer and miR-214-3p was not included in the subsequent analysis.

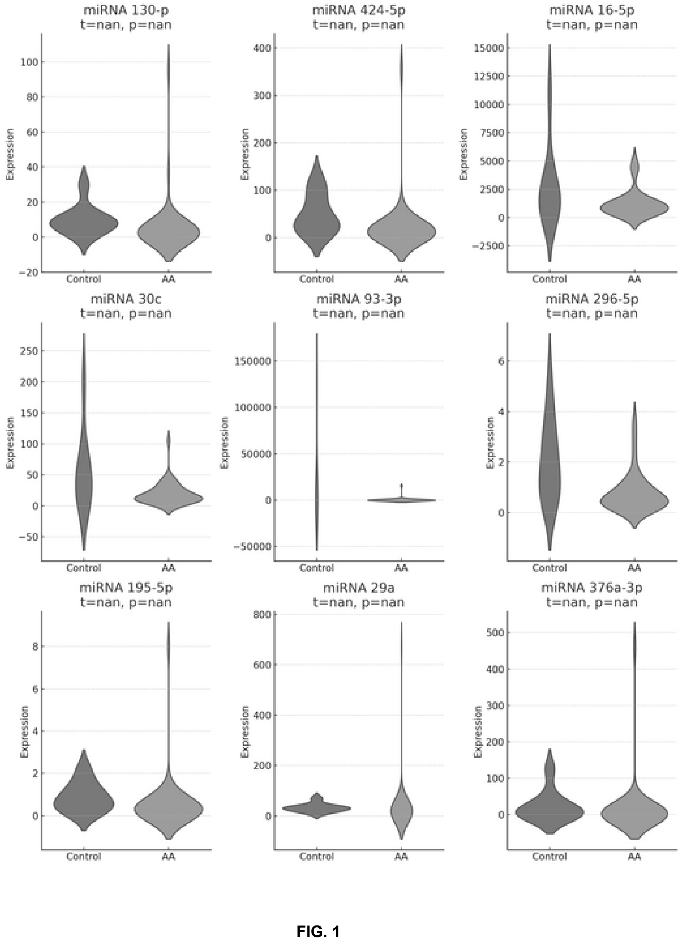

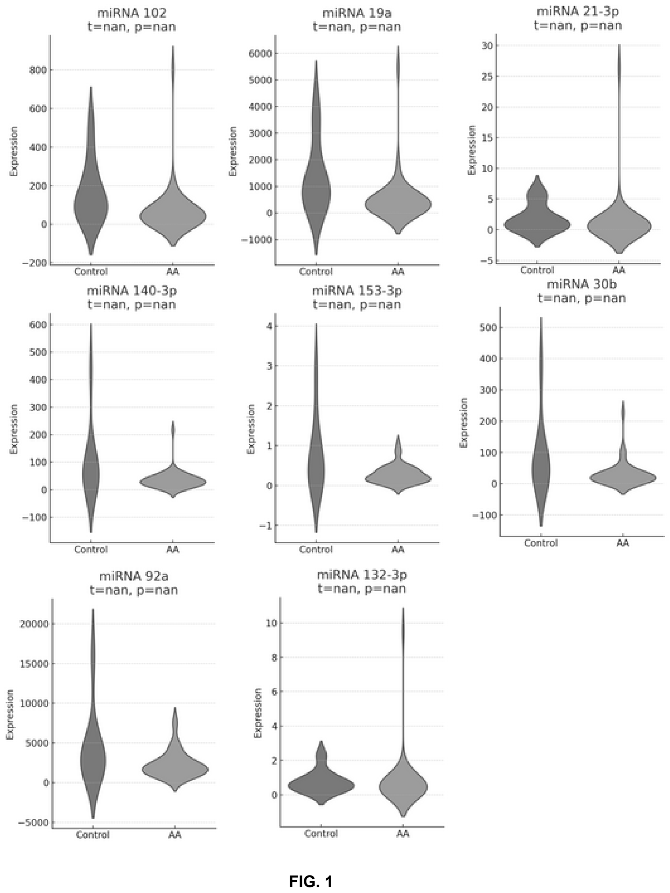

A different population of patients with severe alopecia areata (n=42) and controls (n=13) was used for this validation **(****Figure 1****).** The statistical analysis of the expression values obtained is shown in Table 4.

**Table 4. Statistical analysis of expression levels in plasma samples from patients with severe alopecia areata vs. controls for 17 of the 19 miRNAs in Table 2.**

| **miRNA** | **T-Statistic** | **P value** | **FDR** |
|---|---|---|---|
| **miR 296-5p** | -4.621 | 0.000022 | **0.000131** |
| **miR 19a-3p** | -4.545 | 0.000029 | **0.000131** |
| **miR 30b-5p** | -4.142 | 0.000115 | **0.000278** |
| **miR 101-3p** | -4.121 | 0.000124 | **0.000278** |
| **miR 130b-3p** | -3.652 | 0.000567 | **0.000824** |
| **miR 30c-5p** | -3.613 | 0.000641 | **0.000824** |
| **miR 132-3p** | -3.650 | 0.000570 | **0.000824** |
| **miR 376a-3p** | -3.7032 | 0.000482 | **0.001362** |
| **miR 29b-3p** | -3.5567 | 0.000764 | **0.001443** |
| **miR 424-5p** | -3.5104 | 0.000882 | **0.001499** |
| **miR 140-3p** | -3.3762 | 0.001329 | **0.002053** |
| **miR 195-5p** | -3.0913 | 0.003081 | **0.004364** |
| **miR 21-3p** | -2.9199 | 0.005006 | **0.006547** |
| **miR 92a-3p** | -2.533 | 0.014089 | **0.015850** |
| miR 16-5p | -1.3485 | 0.182800 | 0.194225 |
| miR 93-3p | -1.4339 | 0.157062 | 0.178004 |
| miR 153-3p | -0.976 | 0.333017 | 0.333017 |

| | | | |
|---|---|---|---|
| FDR: false discovery rate. | | | |

This analysis validated the results obtained for 14 (bold) of the 19 previously detected miRNAs that showed differential expression between patients with severe alopecia areata and the control.

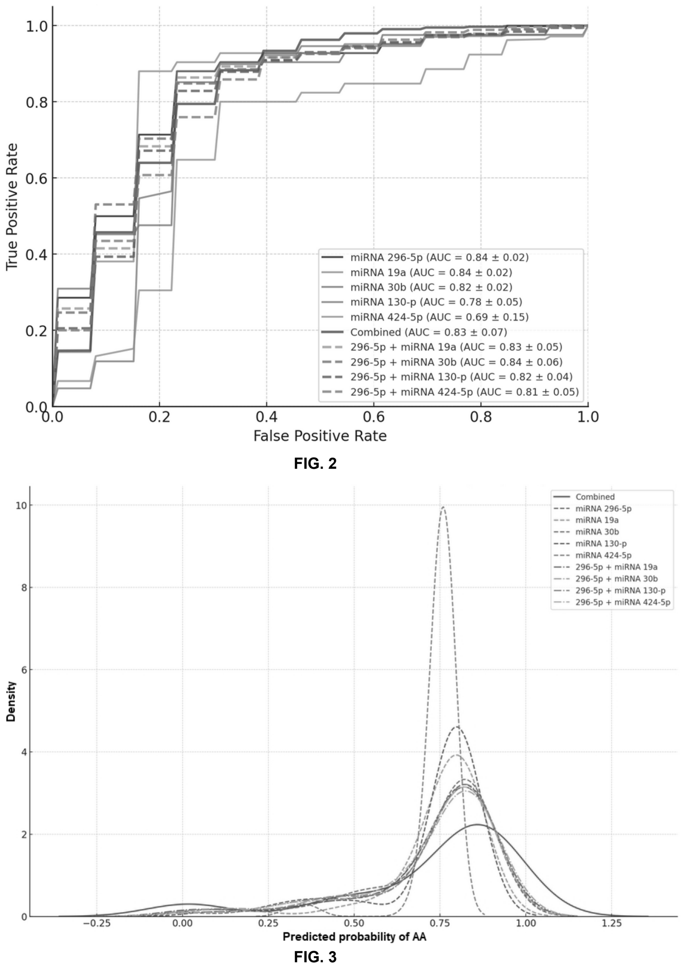

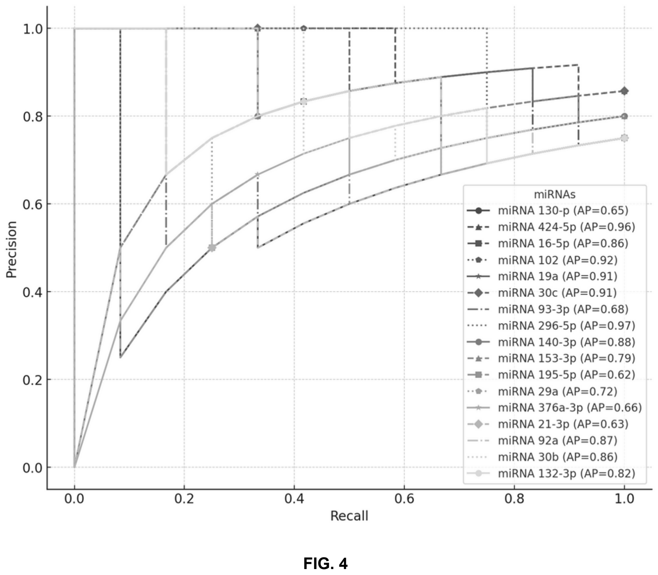

A classification model was developed based on the statistically significant miRNAs (bold) in this validation, the results of which are shown in **Figures 2 to 4** and **Table 5.** The average sensitivity, specificity, and AUC results were calculated for each of the miRNAs, based on a bootstrap analysis with a reduced number of iterations (1000 iterations). The combinations of miRNAs showed an increase in the sensitivity and specificity of the classifications, so different groups of combined miRNAs were tested.

**Table 5: Accuracy analysis (sensitivity and specificity) for the main miRNAs and their combinations.**

| **miRNA/ Combination** | **AUC** | **Sensitivity** | **Specificity** | **Accuracy-Recall AP** | **Accuracy-Cross-validation** |
|---|---|---|---|---|---|
| miR 19a-3p + miR 130b-3p + miR 30b-5p + miR 296-5p + miR 424-5p | 0.872 | 0.929 | 0.769 | 0.93 | 0.859 |
| miR 296-5p | 0.844 | 0.881 | 0.769 | 0.97 | 0.795 |
| miR 19a-3p | 0.841 | 0.881 | 0.846 | 0.91 | 0.747 |
| miR 30b-5p | 0.821 | 0.881 | 0.769 | 0.86 | 0.764 |
| miR 130b-3p | 0.783 | 0.857 | 0.769 | 0.65 | 0.781 |
| miR 424-5p | 0.743 | 0.881 | 0.692 | 0.96 | 0.747 |
| miR 296-5p + miR 19a-3p | 0.857 | 0.905 | 0.769 | 0.94 | 0.796 |
| miR 296-5p + miR 30b-5p | 0.868 | 0.857 | 0.846 | 0.94 | 0.779 |
| miR 296-5p + miR 130b-3p | 0.844 | 0.905 | 0.769 | 0.92 | 0.779 |
| miR 296-5p + miR 424-5p | 0.846 | 0.857 | 0.769 | 0.93 | 0.778 |

As shown in **Table 5 and** **Figures 2 to 4****,** the combination of miRNAs that showed the highest area under the curve (AUC = 0.872) and the highest sensitivity (0.929) was **miR 19a-3p + miR 130b-3p + miR 30b-5p + miR 296-5p + miR 424-5p.**

This combination of miRNAs was verified using other learning methods, obtaining an average accuracy between 0.795 and 0.810.

**Table 6: Validation of the sensitivity and specificity data obtained for the combination of miRNAs miR 19a-3p + miR 130b-3p + miR 30b-5p + miR 296-5p + miR 424-5p using other methods.**

| **Model/Technique** | **Average accuracy** |
|---|---|
| Best Monte Carlo Combination | 0.795 |
| Random Forests | 0.826 |
| Support Vector Machines (SVM) | 0.810 |
| Neural Network | 0.795 |
| Assembly (Voting Classifier) | 0.810 |

Of these methods, Random Forests showed the best individual performance, closely followed by Assembly (Voting Classifier) and SVM. The Best Monte Carlo Combination and Neural Network had similar performance.

To rank the five selected miRNAs (miR 296-5p, miR 19a, miR 30b, miR 130-p, miR 424-5p) according to their individual accuracy within the model, each miRNA was evaluated individually using the model that had the best overall performance, Random Forest. The accuracy of each miRNA was evaluated individually to see how it performed on its own in the classification. This allowed them to be ranked according to their individual predictive ability.

**Table 7: miRNAs ranked by their average accuracy determined using the Random Forest method.**

| | | |
|---|---|---|
| 1. | **miR 19a-3p** | Average accuracy of 0.735 |
| 2. | **miR** 130b-3p | Average accuracy of 0.718 |
| 3. | **miR** 30b-5p | Average accuracy of 0.655 |
| 4. | **miR 296-5p** | Average accuracy of 0.624 |
| 5. | **miR 424-5p** | Average accuracy of 0.623 |

miR 19a-3p had the best individual performance in the classification, followed by miR 130b-3p, miR 30b-5p, miR 296-5p, and miR 424-5p, in that order. This analysis suggests that some miRs may have greater individual predictive power than others. However, it is observed that the highest predictive power is obtained from the combination of the five miRNAs (0.826).

### Specificity of miRNAs

An additional analysis was performed to determine the specificity of the 5 selected miRNAs (miR 19a-3p, miR 130b-3p, miR 30b-5p, miR 296-5p, and miR 424-5p) with respect to other dermatological and autoimmune diseases. For this purpose vitiligo was selected as another inflammatory skin disease with which it shares notable similarities in its pathogenesis, involving both the innate and adaptive immune systems. The expression levels of these markers were compared in isolated peripheral blood samples from patients with severe alopecia areata compared to patients with vitiligo and healthy controls.

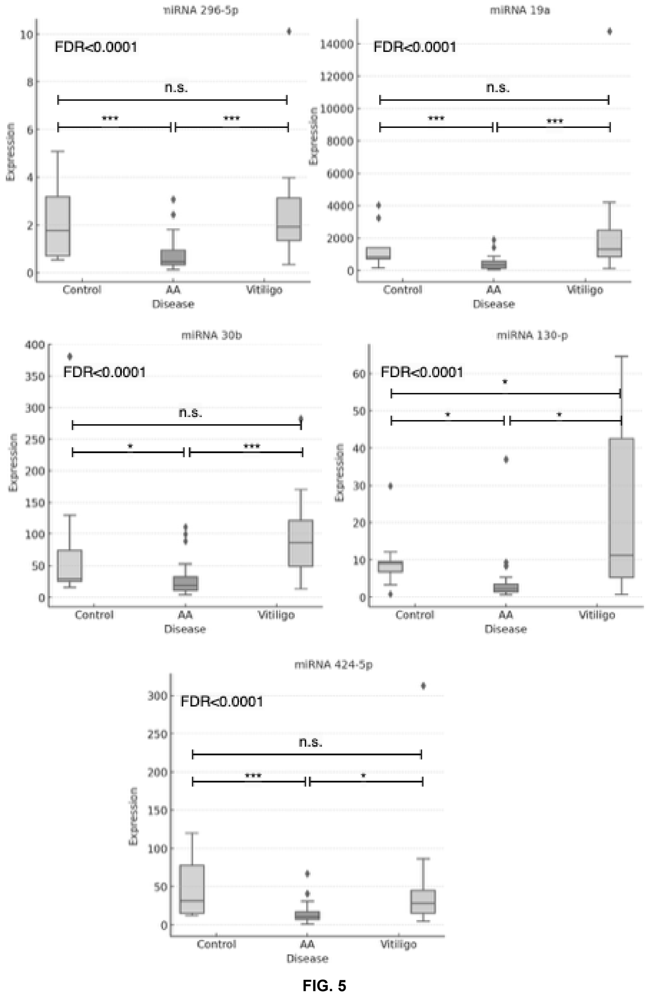

Expression levels were lower in alopecia areata compared to healthy controls and vitiligo controls **(****Figure 5** and **Table 8),** demonstrating that these miRNAs are specific to alopecia areata and not to other inflammatory skin diseases.

**Table 8: Kruskal-Wallis analysis (all FDR adjusted to p-value < 0.0001).**

| **miRNA** | **Comparison** | **Mean Difference** | **p-value** | **Lower CI** | **Upper CI** |
|---|---|---|---|---|---|
| miR 296-5p | Alopecia areata vs. Control | 1.4206 | 0.0010 | 0.6533 | 2.1879 |
| miR 296-5p | Alopecia areata vs. Vitiligo | 1.3539 | 0.0010 | 0.6693 | 2.0384 |
| miR 19a | Alopecia areata vs. Control | 978.9142 | 0.0025 | 306.6531 | 1651.1753 |
| miR 19a | Alopecia areata vs. Vitiligo | 1065.5020 | 0.0010 | 466.0954 | 1664.9086 |
| miR 30b | Alopecia areata vs. Control | 59.6672 | 0.0172 | 8.98 | 110.3545 |
| miR 130-p | Alopecia areata vs. Vitiligo | 19.8614 | 0.0010 | 11.1081 | 28.6147 |
| miR 130-p | Alopecia areata vs. Control | 13.5967 | 0.0217 | 1.6717 | 25.5216 |
| miR 424-5p | Alopecia areata vs. Control | 35.1711 | 0.0010 | 17.0816 | 53.2606 |
| miR 424-5p | Alopecia areata vs. Vitiligo | 16.8463 | 0.0387 | 0.7172 | 32.9754 |

## Claims

1. *In vitro* use of the expression levels of miR 19a-3p, miR 130b-3p, miR 30b-5p, miR 296-5p, and miR 424-5p as biomarkers for diagnosing and/or prognosing severe alopecia areata.

2. The use according to the previous claim, **characterized in that** it also comprises the expression levels of one or more of the following miRNAs: miR 101-3p, miR 30c-5p, miR 132-3p, miR 376a-3p, miR 29b-3p, miR140-3p, miR 195-5p, miR 21-3p, and miR 92a-3p.

3. The use according to the previous claim, **characterized in that** it comprises the expression levels of all miRNAs simultaneously.

4. An *in vitro* method for obtaining useful data for diagnosing and/or prognosing severe alopecia areata, comprising measuring the expression levels of miR 19a-3p, miR 130b-3p, miR 30b-5p, miR 296-5p, and miR 424-5p in a previously isolated biological sample.

5. The *in vitro* method for obtaining useful data according to the previous claim, **characterized in that** the expression levels of one or more of the following miRNAs are also measured: miR 101-3p, miR 30c-5p, miR 132-3p, miR 376a-3p, miR 29b-3p, miR140-3p, miR 195-5p, miR 21-3p, and miR 92a-3p.

6. The *in vitro* method for obtaining useful data according to the previous claim, **characterized in that** the expression levels of all miRNAs are measured simultaneously.

7. An *in vitro* method for diagnosing and/or prognosing severe alopecia areata comprising:
a) measuring the expression levels of miR 19a-3p, miR 130b-3p, miR 30b-5p, miR 296-5p, and miR 424-5p in a previously isolated biological sample,
b) comparing the values obtained with those of a reference sample obtained from healthy control individuals, and
c) classifying the individual in the group of individuals suffering from severe alopecia areata when the miRNAs in step a) are differentially down-regulated compared to samples from healthy control individuals.

8. The *in vitro* method according to the previous claim, **characterized in that** in step a) the expression levels of one or more of the following miRNAs are also measured: miR 101-3p, miR 30c-5p, miR 132-3p, miR376a-3p, miR 29b-3p, miR140-3p, miR 195-5p, miR 21-3p, and miR 92a-3p.

9. The *in vitro* method according to the previous claim, **characterized in that** in step a) the expression levels of all miRNAs are measured simultaneously.

10. The method according to any of claims 4 to 9, **characterized in that** the previously isolated biological sample is selected from blood, plasma, and serum.

11. The method according to the previous claim, **characterized in that** the biological sample is plasma.

12. The method according to any of claims 4 to 11, wherein said result can be obtained by:
(i) a method of generating miRNA profiles, such as a microarray, and/or
(ii) a method comprising PCR (polymerase chain reaction), such as real-time PCR or RT-PCR, and/or
(iii) a Northern Blot and/or
(iv) an immunoassay, such as an immunohistochemistry method or an ELISA-based method.

13. A kit or device comprising at least one or more oligonucleotides capable of hybridizing with miR 19a-3p, miR 130b-3p, miR 30b-5p, miR 296-5p, and miR 424-5p under stringent conditions.

14. Use of the kit according to the previous claim in a method according to any of claims 4 to 12.

15. Use of miR 19a-3p, miR 130b-3p, miR 30b-5p, miR 296-5p, and miR 424-5p miRNAs in a method for predicting therapeutic response to treatment for severe alopecia areata.
